# FASCICULE DE BREVET EUROPEEN

(11) **EP 3 191 080 B1**
(45) Date de publication et mention de la délivrance du brevet: **20.03.2019**
(21) Numéro de dépôt: 15759475.5
(22) Date de dépôt: 09.09.2015
(51) Int. Cl.: A61K 9/08, A61K 45/06, A61K 31/167, A61K 31/192, A61K 31/7048, A61K 9/00, A61K 47/20, A61K 31/165, A61K 31/5415

(54) **COMPOSTIONS PARENTÉRALES ET LEURS UTILISATIONS**
PARENTERALE ZUSAMMENSETZUNGEN UND IHRE ANWENDUNGEN
PARENTERAL COMPOSITIONS AND USES THEREOF

(30) Priorité: 09.09.2014 EP 14306385
(43) Date de publication de la demande: 19.07.2017
(73) Titulaire: Ceva Santé Animale, 33500 Libourne Cedex (FR)
(72) Inventeur: GUIMBERTEAU, Florence, F-33450 Montussan (FR); GENETEAU, Anne, F-33500 Les Billaux (FR); GUYONNET, Jérôme, F-33440 Ambares (FR)
(74) Mandataire: Cabinet Becker et Associés
(86) Numéro de dépôt international: PCT/EP2015/070539
(87) Numéro de publication internationale: WO 2016/038059

(56) Documents cités:
- WO-A1-2012/099479
- STRICKLEY R G: "SOLUBILIZING EXCIPIENTS IN ORAL AND INJECTABLE FORMULATIONS", PHARMACEUTICAL RESEARCH, SPRINGER NEW YORK LLC, US, vol. 21, no. 2, 1 février 2004 (2004-02-01), pages 201-230, XP009035738, ISSN: 0724-8741, DOI: 10.1023/B:PHAM.0000016235.32639.23

## Description

La présente invention a pour objet une composition parentérale comprenant un antibiotique et un agent anti-inflammatoire non stéroïdien en solution dans un solvant particulier, selon la revendication 1. La composition est en particulier destinée à être injectée chez un mammifère non-humain, en particulier un animal d'élevage. Elle a trait également à son utilisation dans un procédé pour traiter des désordres liés à une infection microbienne chez des mammifères non-humains par injection de ladite composition.

### Arrière-plan technologique

La voie parentérale (comme la voie sous-cutanée, intramusculaire ou intraveineuse) est privilégiée pour administrer des substances à des mammifères non-humains, en particulier les animaux d'élevage comme par exemple les bovins.

Les injections intramusculaires (IM) sont utilisées pour les médicaments (antibiotiques, anti-inflammatoires...), certains antiparasitaires, les toniques généraux, certaines vitamines et oligo-éléments, les hormones, les anesthésiques, les analgésiques et les vaccins. Elles sont réalisées principalement dans les masses musculaires de l'encolure, où les risques d'abcès sont plus faibles et la résorption est meilleure en raison des mouvements permanents de l'encolure. Les substances sont absorbées au niveau des vaisseaux sanguins du tissu conjonctif qui emballe les fibres musculaires. Il est également possible de piquer dans les muscles du plat de la fesse ou dans les muscles de l'arrière de la cuisse. Le volume maximal par point d'injection est généralement de 25 à 30 mL pour un bovin adulte, 10 à 20 mL pour un jeune bovin et 5 à 10 mL pour un veau. Si la quantité à injecter dépasse ces volumes, les points d'injection sont multipliés, par exemple de part et d'autre de l'encolure, afin d'assurer une résorption rapide et totale de la substance. L'injection intramusculaire doit se faire à une vitesse contrôlée pour ne pas dilacérer les muscles.

Les injections sous-cutanées (SC) sont utilisées pour l'administration de sérums (exemple : sérum anti-tétanique), de vaccins, de certains antiparasitaires internes, d'antibiotiques et anti-inflammatoires. Les injections sous-cutanées sont effectuées en avant de l'épaule ou sur le plat de l'encolure. Le produit est déposé dans le tissu conjonctif sous-cutané où il diffuse lentement. Sa résorption est assurée par les vaisseaux sanguins. La quantité de liquide injectée par voie SC est généralement au maximum de 50 mL chez un bovin adulte, 20 mL chez un jeune et 10 mL chez un veau. Si le volume à injecter est plus important, les injections seront réparties en plusieurs points.

Les injections intraveineuses (IV) sont réalisées généralement à la veine jugulaire de l'encolure, plus rarement dans la veine mammaire. Il est possible, pour les faibles quantités à injecter (moins de 20mL) et sur des gros animaux (plus de 300 kg), d'injecter dans la veine ou l'artère coccygienne médiale, sous la queue, au niveau de la 3ème vertèbre coccygienne. L'injection intraveineuse doit toujours être pratiquée lentement, avec des produits idéalement portés à la température de 20-30°C. Si des signes d'intolérance apparaissent (tremblements, salivation, grincements de dent...), l'injection doit être interrompue ou poursuivie encore plus lentement.

L'injection de substances à des mammifères non-humains comme par exemple un bovin doit se faire correctement afin d'éviter toute blessure à l'animal. En général, elle est réalisée par l'éleveur et/ou le vétérinaire. En outre, l'injection parentérale peut entraîner des réactions inflammatoires locales au point ou autour du site d'injection qui se traduisent par l'apparition de douleurs et de souffrance, pouvant générer une incapacité physique partielle ou totale de l'animal.

Des injections parentérales mal ou partiellement réalisées se traduisent par une mauvaise absorption des substances entraînant des problèmes d'observance du traitement ou la présence de résidus de médicaments dans l'animal traité qui aura comme conséquence de retarder ou de rendre impropre à la consommation sa viande ou son lait.

Une difficulté dans la mise au point de ces compositions parentérales réside principalement dans le manque de connaissance de mécanismes généraux de pénétration des substances à travers l'épiderme et les couches sous-jacentes de la peau. Il est donc particulièrement difficile de prédire quels composés peuvent être délivrés par voie parentérale et quels seront les solvants adaptés à ce mode d'administration.

En outre, la nature de la peau des mammifères non-humain explique pourquoi très peu, voire aucun, des produits transdermiques disponibles dans le commerce pour un usage humain n'est efficace.

En conséquence, il persiste aujourd'hui, dans le domaine de la santé animale et plus particulièrement dans le domaine des médicaments injectables, un besoin pour de nouvelles compositions liquides et stables et procédé d'administration parentérale liquide et stable, qui offrent à l'utilisateur un moyen sûr et commode d'administrer un médicament tout en minimisant ses résidus et en réduisant fortement la douleur ou le stress de l'animal.

Chez les bovins, le terme général de « maladies respiratoires » désigne un ensemble de troubles respiratoires qui affectent les voies respiratoires basses, c'est-à-dire les poumons (pneumonie) ou les voies respiratoires hautes (rhinite, trachéite, bronchite). Ces maladies sont généralement causées par divers agents pathogènes et notamment des bactéries Gram négatif comme *Pasteurella multocida, Mannheimia haemolytica, Histophilus somni, Mycoplasma bovis.* Ces agents interagissent les uns avec les autres ou conjointement avec un processus inflammatoire ou une réaction allergique pour déclencher la maladie proprement dite. Les principaux signes cliniques observés sont la fièvre, la léthargie, un manque d'appétit et un abattement lié à des signes respiratoires plus ou moins marqués (respiration superficielle et rapide, légère toux, écoulement nasal et oculaire de type aqueux et salivation importante). Ces maladies peuvent entraîner des pertes économiques importantes dans les exploitations affectées principalement dues à la diminution du poids corporel des animaux, à l'augmentation de la mortalité et aux coûts associés au travail supplémentaire (séparation des animaux infectés, traitement). Le traitement doit cibler la cause de la maladie et les signes cliniques. Pour ce faire, il est usuel d'administrer de façon séquentielle ou concomitante divers traitements spécifiques comme des antibiotiques, des antiparasitaires, des anti-inflammatoires non stéroïdiens, des bronchodilatateurs et des mucolytiques.

La mammite est une réaction inflammatoire de la glande mammaire principalement d'origine bactérienne. Sa prévalence est élevée parmi les vaches laitières et elle représente l'une des maladies les plus importantes dans l'industrie laitière. Les principaux agents pathogènes responsables des mammites sont des bactéries Gram positif comme *Staphylococcus aureus, Streptococcus uberis* et des Gram négatif, comme *Escherichia coli* et chlamydia ainsi que les mycoplasmes. Une fois que les bactéries ont infecté la mamelle, il est nécessaire d'éliminer les bactéries rapidement et de manière efficace en utilisant un traitement antibiotique par voie générale adapté au cas clinique et faire diminuer l'inflammation à l'aide d'un anti-inflammatoire.

D'autres mammifères non-humains, à l'instar des bovins, présentent des pathologies d'origine bactérienne accompagnés par des signes cliniques inflammatoires qui nécessitent l'administration de façon séquentielle ou concomitante de traitements antibiotiques et d'anti-inflammatoires, en particulier des anti-inflammatoires non stéroïdiens.

Aujourd'hui, environ 50 antibiotiques ont reçu une autorisation de mise sur le marché (AMM). Ils sont répartis en 11 familles, que sont les aminosides, les bêtalactamines (pénicillines / céphalosporines), les phénicolés, les tétracyclines, les macrolides (et apparentés), les polypeptides, les sulfamides, les quinolones, les nitro-imidazolés, les dérivés des nitrofuranes et les dérivés du noyau benzyl-pyrimidine. Les antibiotiques sont généralement des agents bactéricides ou bactériostatiques orientés contre les bactéries à Gram positif, comme par exemple *Staphylococcus* et *Streptococcus,* contre les bactéries à Gram négatif, comme par exemple *E. Coli, Salmonella, Chlamydia, Citrobacter, Enterobacter, Serratias, Morganella, Proteus, Klebsiella, Shigella, Pasteurella, Mannheimia*, *Haemophilus, Moraxella, Pseudomonas, Brucella*) ou contre les mycoplasmes, comme par exemple *mycoplasma.*

Les anti-inflammatoires sont destinés à traiter une réaction inflammatoire et les maladies qui en résultent. Il existe principalement deux grandes familles d'anti-inflammatoire : les glucocorticoïdes et anti-inflammatoires non stéroïdiens (AINS). Les AINS sont des médicaments aux propriétés analgésiques, antipyrétiques et/ou anti-inflammatoires.

De nombreux antibiotiques sous forme de compositions parentérales liquides (transdermale) sont disponibles dans le commerce. On peut citer comme exemple un antibiotique phénicolé, tel que le florfenicol commercialisé par la société Schering-Plough sous la marque Nuflor® décrite dans le brevet US5,082,863, ou des formes injectables en intramusculaire décrites dans la demande internationale WO2004/014340 et la demande US2003/0068339.

La demande de brevet WO2009/156369 décrit des compositions transdermales liquides comprenant un agent anti-inflammatoire non stéroïdien (AINS) et un support acceptable sur le plan pharmaceutique comprenant un solvant et au moins un activateur de pénétration cutanée. Cette demande de brevet illustre des compositions comprenant du méloxicam, le kétoprofène et l'acide tolfenamique avec un système de solvant caractérisé par un activateur de pénétration cutanée qui est un mélange de L-menthol et de miglyol 840, et un solvant qui est soit du diéthylène glycol monoéthyl éther (DEGMEE), soit un mélange d'isopropyl alcool avec du 2-pyrolidone ou du N-méthyle pyrolidone. Cette composition transdermique liquide d'un AINS peut contenir optionnellement un antibiotique.

Cependant, aucun résultat présenté dans cette demande de brevet ne permet de surmonter les problèmes d'interactions pharmacocinétiques, d'absorption, de distribution, de biotransformation et/ou d'excrétion bien connu par l'homme du métier qui se produit lors de l'administration d'un AINS et d'un antibiotique, tel que décrit dans Benet L.and al. 1996: Pharmacological Basis of Therapeutics. pp. 3-28 et dans Chaudary and al 1999 : Buffalo Bull. 18, 27-30 et Sharma et al. 2012 : Vet. Archiv. 82, 555-565.

C'est donc afin de remédier aux problèmes d'interactions rencontrées avec l'association d'anti-inflammatoires non stéroïdiens (AINS) et d'antibiotiques, et de proposer des compositions concentrées de sorte à en réduire le volume d'application, d'améliorer le bien être du mammifère non-humain en limitant le nombre de points d'injections, tout en conservant une bonne stabilité lors du stockage, et surtout l'absence de formation de cristaux, que la Demanderesse a mis au point une composition qui fait l'objet de l'Invention. Elle s'est en particulier donné pour but de proposer un produit visant le traitement préventif et curatif des infections bactériennes en agissant simultanément sur la cause de la maladie et les signes cliniques.

Un autre objectif de l'invention est de fournir de telles compositions facilement administrables en un seul point d'injection quelle que soit l'espèce animale et sa taille.

Encore un autre objectif de l'invention est de fournir en une seule composition liquide parentérale un mélange d'antibiotique et d'AINS, sans rencontrer de problèmes d'interactions biopharmaceutiques entre ces substances.

A ce titre, la Demanderesse a découvert que la solubilisation d'un AINS et d'un antibiotique, plus particulièrement d'un antibiotique phénicolé et d'un oxicam, et encore plus préférentiellement du florfenicol et du méloxicam, à l'aide d'une proportion suffisante de solvant diméthylsulfoxyde (DMSO) permettait de résoudre de manière efficace les problèmes rencontrés de l'art antérieur. En effet, il a été découvert de manière surprenante que la formulation d'un AINS et d'un antibiotique, plus particulièrement d'un antibiotique phénicolé et d'un oxicam, avec au moins 35% de DMSO, permettait de solubiliser de plus fortes concentrations d'actifs sans risque de recristallisation. L'ajout du DMSO présente aussi l'avantage de ne pas avoir à prévoir des conditions spéciales pour le stockage, telles que des réserves à températures modérées au dessus du point de congélation. Ceci est particulièrement surprenant puisque le DMSO n'est généralement pas choisi en tant que solvant pour des formulations liquides du fait de son point de congélation élevé proche de 18°C.

### Résumé de l'invention

La présente invention propose une composition vétérinaire comprenant le florfénicol, au moins un anti-inflammatoire non stéroïdien (AINS), au moins 35% de diméthylsulfoxyde, et du glycérol formal, destinée à être appliquée de manière parentérale à des mammifères non-humains, en particulier des animaux d'élevage et/ou des animaux domestiques.

La composition parentérale selon la présente invention présente une excellente stabilité. Elle est également pratique à administrer tout en minimisant les résidus d'actifs et/ou en réduisant la douleur et/ou le stress de l'animal.

L'invention concerne également l'utilisation de cette composition dans un procédé de traitement de désordres liés à une infection microbienne de mammifères non-humains, en particulier d'animaux d'élevage, par injection de ladite composition.

### Description détaillée de l'invention

La présente invention a pour objet une composition parentérale comprenant le florfénicol, un anti-inflammatoire non stéroïdien (AINS) et au moins 35% de diméthylsulfoxyde (DMSO), ainsi que du glycérol formai.

La composition selon l'invention est avantageusement sous forme liquide.

Dans la présente description, les pourcentages sont exprimés en poids (g) par volume (100ml) de la composition, sauf mention contraire. Ainsi, 35 % signifie que le DMSO est présent en une quantité de 35 g pour 100ml de la composition.

La composition comprend de préférence au moins 40%, ou 45%, ou 50% ou 55% ou 60% de DMSO.

Les anti-inflammatoires non-stéroïdiens (AINS) sont constitués notamment de l'acide acétylsalicylique et acétylsalicylate de lysine, de salicylés comme notamment le salicylate de méthyle, de dérivés arylacétiques, ou arylalkanoïques, comme par exemple le diclofénac, l'acéclofénac, le sulindac ou le kétorolac (trométamol), d'acides 2-arylpropioniques (profènes), comme par exemple l'ibuprofène, le kétoprofène, le dexkétoprofène, le naproxène, l'oxaprozine et le flurbiprofène, de dérivés indoliques, comme par exemple l'indométacine (ou indométhacine) ou la proglumétacine, d'oxicams, comme par exemple le méloxicam, le piroxicam et le ténoxicam, d'inhibiteurs de cyclo-oxygénase donneurs de monoxyde d'azote (Cox inhibiteurs), comme par exemple le naproxcinod, de sulfonanilides, d'inhibiteurs sélectifs de la cyclo-oxygénase 2, comme par exemple la célécoxib, l'étoricoxib, le parécoxib, le rofécoxib et le valdécoxib, des phénylbutazone, de l'acide niflumique et des acides N-arylanthraniliques (acides phénamiques).

De préférence, la présente invention a pour objet une composition parentérale comprenant le florfénicol, un AINS de la famille des oxicams et au moins 35% de DMSO. La composition comprend également du glycérol formal. L'antibiotique dans la présente invention est le florfénicol.

Parmi les agents anti-inflammatoires non-stéroïdiens de la famille des oxycams, on peut citer en particulier le méloxicam, piroxicam ou le tenoxicam. Selon un mode spécifique, l'agent anti-inflammatoire non-stéroïdien de la famille des oxycams est le méloxicam.

De façon encore plus préférentielle, la présente invention a pour objet une composition parentérale comprenant du florfénicol, du méloxicam et au moins 35% de DMSO.

La composition selon l'invention peut comprendre éventuellement d'autres solvants polaires aprotiques On peut citer à titre d'exemples de solvants polaires aprotiques choisi parmi les alcools, cétones telles que l'acétone et la butanone, parmi les amines N, N disubstituées telles que le diméthylformamide (DMF), N-méthyl-2-pyrrolidone (NMP) parmi les nitriles tels que l'acétonitrile, parmi les esters tels l'acétate d'éthyle, l'acétate de propyle, ou l'acétate d'amyle, parmi les amines tertiaires telles que le triéthylamine, parmi les hétérocycles azotés, tels que la pyridine, ou parmi les dioxolanes, tel que le glycérol formai.

La composition selon l'invention comprend deux solvants, plus spécifiquement le DMSO et le glycérol formal.

De préférence, l'antibiotique (plus spécifiquement le florfénicol) est généralement présent dans la composition selon l'invention en une proportion comprise entre 20 et 60 %, de préférence entre 30 et 50%, ou encore entre 35 et 45%, et plus spécifiquement de 40%.

L'anti-inflammatoire non-stéroïdien (plus spécifiquement le méloxicam) est généralement présent dans la composition selon l'invention en une proportion pouvant varier de 0,1 à 1%, de préférence entre 0,2 et 0,8 %, et mieux entre 0,4 et 0,6 %.

D'une manière générale, les compositions selon l'invention sont également appelées médicaments ou compositions vétérinaires ou pharmaceutiques antibiotiques.

Le terme « comprend » dans la présente invention peut être interprété de manière plus stricte ; il peut ainsi être remplacé selon des modes particuliers par les termes « consiste essentiellement en » ou encore par « consiste en ».

Ces compositions vétérinaires peuvent en outre comprendre un solvant physiologiquement acceptable et/ou un excipient également physiologiquement acceptable. Par physiologiquement acceptable, on entend un solvant ou un excipient qui ne soit pas nocif pour les animaux destinés à recevoir la composition selon l'invention. Ainsi, comme décrit précédemment, les compositions selon l'invention peuvent comprendre un solvant, un régulateur de pH, un agent tampon, un agent de mise en suspension, un agent de solubilisation, un stabilisant, un agent de tonicité, un antioxydant et/ou un conservateur.

Comme autre solvant, on peut citer les solvants huileux tels que les triglycérides à chaîne moyenne en C₈-C₁₀, ou un mélange d'acide caprique, d'acide caprylique et de triglycérides, tels que ceux qui sont commercialisés sous la désignation Mygliol®812, l'huile de sésame, le dicaprylocaprate de propylène glycol, l'éthyle oléate.

Des exemples d'agents de mise en suspension englobent la méthylcellulose, le polysorbate 80, les esters de sorbitan, l'hydroxyéthylcellulose, la gomme de xanthane, la carboxyméthycellulose sodique et le monolaurate de sorbitane polyéthoxylé. Des exemples d'agent de solubilisation englobent l'huile de ricin solidifiée par du polyoxyéthylène, le polysorbate 80, le nicotinamide, le monolaurate de sorbitane polyéthoxylé, le macrogol et l'ester éthylique d'acide gras d'huile de ricin. En outre, le stabilisant englobe le sulfite de sodium, le métalsulfite de sodium et l'éther, l'éthylène diamine tétra acétique, tandis que le conservateur englobe le p-hydroxybenzoate de méthyle, le p-hydroxybenzoate d'éthyle, l'acide sorbique, l'alcool benzylique, le phénol, le crésol et le chlorocrésol. Parmi les antioxydants, on peut citer à titre d'exemples non limitatif : l'hydroxytoluène butylé, l'hydroxyanisole butylé, la vitamine E, le tert-butylhydroquinone et l'acide citrique. Un exemple d'agent de tonicité est le mannitol.

Lors de la préparation des solutions ou suspensions injectables, il est souhaitable de veiller à ce qu'elles soient isotoniques au sang.

Les proportions des différents éléments entrant dans les compositions vétérinaires selon l'invention sont déterminées par les pratiques standards bien connues de l'homme du métier spécialisé dans la galénique des produits pharmaceutiques et vétérinaires.

De préférence, les compositions vétérinaires selon l'invention sont administrées par injection, en particulier par injection intramusculaire ou sous-cutanée. Elles se présentent en général sous la forme de solutions ou de suspensions liquides. Ces compositions antibiotiques peuvent être conservées pendant plusieurs mois, à savoir 1 mois, 2 mois, 3 mois jusqu'à 6 mois à température ambiante ou à une température plus élevée allant jusqu'à 40°C, et gardent un aspect limpide et clair sans dégradation ni formation de précipité du ou des principes actifs et plus particulièrement du florfénicol et du méloxicam

De manière avantageuse, les compositions vétérinaires peuvent être administrées en une seule ou plusieurs doses injectables.

Les compositions injectables sont préparées par simple mélange des composants indiqués ci-dessus.

La présente invention a en outre pour objet un kit à usage vétérinaire en particulier destiné à traiter par injection des mammifères non-humains, en particulier des animaux d'élevage, contre les désordres liés à une infection microbienne. Les kits selon la présente invention comportent au moins un compartiment pour un conditionnement éventuellement stérile et comprenant la composition selon l'invention. Le kit comprend également les moyens permettant l'administration des compositions par injection, en particulier par injection intramusculaire ou sous-cutanée, tels que notamment au moins une seringue et au moins une aiguille, et éventuellement une fiche d'instructions concernant le mode d'utilisation des compositions vétérinaires selon l'invention.

La composition selon l'invention est en particulier destinée à être injectée chez un mammifère non-humain, en particulier un animal d'élevage ou de compagnie.

Elle est particulièrement utile pour un traitement des désordres liés aux infections microbiennes de mammifères non-humains, en particulier d'animaux d'élevage ou de compagnie, par injection de ladite composition.

Les animaux d'élevage ou de compagnie sont en particulier les bovins, porcins, ovins, canidés ou félins, plus particulièrement les bovins.

Les animaux d'élevage sont plus spécifiquement destinés à la consommation (pour la viande, les abats ou le lait). Ils sont en particulier les bovins, les porcins ou les ovins. Selon un mode particulier, les animaux d'élevage sont des boeufs, des vaches (pour lactation ou non) ou des cochons.

Les animaux domestiques sont plus spécifiquement des animaux de compagnie comme par exemple les canidés et les félins.

Les désordres liés aux infections microbiennes, notamment bactériennes, peuvent être en particulier les maladies respiratoires qui affectent les voies respiratoires basses, c'est-à-dire les poumons (pneumonie) ou les voies respiratoires hautes (rhinite, trachéite, bronchite), qui peuvent toucher les mammifères non-humains, et en particulier les animaux d'élevage, comme les bovins ou les ovins.

Chez les bovins plus spécifiquement, le terme général de « maladies respiratoires » désigne un ensemble de troubles respiratoires.

Le traitement des infections microbiennes permettent de traiter de manière préventive ou curative des maladies respiratoires, en particulier des bovins, ou les mammites, en particulier celles des bovins, et plus spécifiquement des vaches en lactation.

D'une manière générale, en thérapeutique animale, le vétérinaire ou l'éleveur peut déterminer la posologie qu'il estime la plus appropriée en fonction d'un traitement préventif ou curatif, en fonction de l'âge, du poids de l'animal, et des autres facteurs propres au sujet à traiter. Les dosages se font grâce à des systèmes de délivrance et de dosage connus de l'homme du métier.

Enfin, l'invention concerne également la composition pour une utilisation dans un procédé de traitement des désordres liés à des infections microbiennes, en particulier bactériennes, de mammifères non-humains, en particulier d'animaux d'élevage ou domestiques, par injection de la composition selon l'invention.

L'invention est illustrée par les exemples qui suivent, sans en limiter sa portée.

### EXEMPLES

### EXEMPLE 1 : Stabilités de compositions comprenant un agent anti-inflammatoire non-stéroïdien et un antibiotique

### Composition 1 : Solution liquide comprenant du florfenicol et du méloxicam

40g de florfenicol et 0,5 g de méloxicam sont solubilisés dans 45g de dimethylsulfoxyde (DMSO). Du glycérol formal est ajouté pour amener le volume final à 100mL
La solution ainsi obtenue est stable d'un point de vue physico-chimique pendant au moins 3 mois à des températures comprises entre 4 et 40°C.

### Composition 2 : Solution liquide comprenant du florfenicol et du kétoprofène

40 g de florfenicol et 3g de kétoprofène sont solubilisés dans 45g de dimethylsulfoxyde (DMSO). Du glycérol formal est ajouté pour amener le volume final à 100mL. Une solution limpide incolore est ainsi obtenue et est stable d'un point de vue physico-chimique pendant au moins 3 mois à des températures comprises entre 4 et 40°C.

### Composition 3 : Solution liquide comprenant du florfenicol et du piroxicam

40 g de florfenicol et 2.5 g de piroxicam sont solubilisés dans 45g de dimethylsulfoxyde (DMSO). Du glycérol formal est ajouté pour amener le volume final à 100mL. Une solution limpide légèrement colorée en jaune est ainsi obtenue et est stable d'un point de vue physico-chimique pendant au moins 3 mois à des températures comprises entre 4 et 40°C.

### Composition 4 (exemple de référence) : Solution liquide comprenant de la tylosine et du kétoprofène

8 g de tylosine tartrate et 2.4 g de kétoprofène sont solubilisés dans 60 g de dimethylsulfoxyde (DMSO). Du glycérol formal est ajouté pour amener le volume final à 100mL. Une solution limpide légèrement colorée en jaune est ainsi obtenue est stable d'un point de vue physico-chimique pendant au moins 3 mois à des températures comprises entre 4 et 40°C.

### Composition 5 (exemple de référence): Solution liquide comprenant de la tylosine et du kétoprofène

8 g de tylosine tartrate et 2.4 g de kétoprofène sont solubilisés dans 60 g de dimethylsulfoxyde (DMSO). De l'acétate d'éthyle est ajouté pour amener le volume final à 100mL. Une solution limpide légèrement colorée en jaune est ainsi obtenue est stable d'un point de vue physico-chimique pendant au moins 3 mois à des températures comprises entre 4 et 40°C.

### EXEMPLE 2 : Tests d'équivalence de biodisponibilité

24 jeunes bovins males et femelles âgés de 11 mois et pesant 220 kg en moyenne, sont traités individuellement par 1 injection sous cutanée de 40 mg/kg de florfenicol (Nuflor 450® - Merck, 450 mg de florfenicol/mL). Durant les 7 jours suivant cette injection, des prélèvements de sang sont régulièrement réalisés. Suit alors une période de « wash out » de 20 jours. Les animaux reçoivent alors une injection sous cutanée de 0,5 mg/kg méloxicam (Metacam®, Boeheringer Ingelheim, 5 mg de méloxicam/mL). Durant les 7 jours suivant cette injection, des prélèvements de sang sont régulièrement réalisés. Suit alors une nouvelle période de « wash out » de 20 jours. Les animaux reçoivent alors une injection sous cutanée de 1mL/ Kg de la Composition 1 (Florfénicol et méloxicam). Durant les 7 jours suivant cette injection, des prélèvements de sang sont régulièrement réalisés.

Les prélèvements sanguins réalisés durant cette campagne de traitement permettent d'évaluer les paramètres pharmacocinétiques (Cmax et AUC₀₋ₜ) et pharmacologique (temps d'antibiotique utile) par des méthodes classiques connues de l'homme du métier.

### Paramètres pharmacocinétiques :

**Tableau 1 : Détermination du Cₘₐₓ et de l'AUC plasmatique du florfénicol**

| | Florfénicol | Composition 1 |
|---|---|---|
| Cₘₐₓ (µg/L) | 3500 ± 1035 | 4647 ± 1597 |
| AUC₀₋ₜ (h*µg/L) | 196289 ± 44987 | 228139 ± 48084 |

**Tableau 2 : Détermination du Cₘₐₓ et de l'AUC plasmatique du méloxicam**

| | Méloxicam | Composition 1 |
|---|---|---|
| Cₘₐₓ (µg/L) | 2239 ± 357 | 2011 ± 338 |
| AUC₀₋ₜ (h*µg/L) | 40569 ± 7122 | 34605 ± 5860 |

### Paramètre Pharmacologique :

Le temps d'antibiotique utile est la durée (en heure) pendant laquelle la concentration plasmatique d'un antibiotique donné est supérieure à sa CMI₉₀ pour les 3 germes représentatifs *Mannheimia haemolytica, Histophilus somni, Pasteurella multocida.*

Les CMI₉₀ de référence pour le florfénicol sur ces 3 souches de référence sont : 1 µg/mL pour *Mannheimia haemolytica,* 0,2 µg/mL pour *Histophilus somni* et 0,5 µg/mL pour *Pasteurella multocida.*

**Table 3 : Temps antibiotique calculé (exprimé en heures)**

| | Composition 1 | Florfénicol |
|---|---|---|
| *Mannheimia haemolytica* | 72.08 ± 18.23 | 66.44 ± 20.65 |
| *Histophilus somni* | 160.48 ± 14.72 | 167.78 ± 0.13 |
| *Pasteurella multocida* | 120.40 ± 24.78 | 132.03 ± 27.22 |

Les principaux paramètres pharmacocinétiques et pharmacologiques du florfénicol sont comparables, et démontrent ainsi une exposition et une efficacité comparables de l'antibiotique entre la formule contenant du florfénicol ou du méloxicam seul et les compositions selon l'invention.

## Revendications

1. Composition vétérinaire parentérale comprenant un agent anti-inflammatoire non-stéroïdien, un antibiotique qu'est le florfénicol, au moins 35 %, % exprimés en g/100ml, de diméthylsulfoxyde, et au moins un solvant polaire aprotique qu'est le glycérol formai.

2. Composition selon la revendication 1, dans laquelle l'agent anti-inflammatoire non-stéroïdien est de la famille des oxycams, de préférence choisi parmi le méloxicam, piroxicam ou le tenoxicam, et en particulier le méloxicam.

3. Composition selon l'une des revendications précédentes, la composition étant destinée à être injectée chez un mammifère non-humain, en particulier un animal domestique ou d'élevage.

4. Composition selon la revendication 3, **caractérisée en ce que** ledit animal est un bovin, un porcin, un ovin, un canidé ou un félin et en particulier un bovin.

5. Compositions selon la revendication 3, dans laquelle l'injection est intramusculaire ou sous-cutanée.

6. Composition selon l'une des revendications 1 et 2, pour une utilisation dans le traitement des désordres liés à des infections microbiennes de mammifères non-humains, en particulier par injection de ladite composition, dans laquelle les désordres liés à des infections microbiennes sont des troubles ou maladies respiratoires qui affectent les voies respiratoires basses ou hautes.

7. Kit à usage vétérinaire comprenant une composition telle que définie selon l'une des revendications 1 à 6 et au moins un compartiment pour un conditionnement éventuellement stérile.

## Patentansprüche

1. Parenterale tiermedizinische Zusammensetzung umfassend ein nicht-steroidales entzündungshemmendes Mittel, ein Antibiotikum, das Florfenicol ist, wenigstens 35% Dimethylsulfoxid, wobei die Prozentangabe für g/100 ml Zusammensetzung steht, und wenigstens ein aprotisches polares Lösemittel, das Glycerinformal ist.

2. Zusammensetzung gemäß Anspruch 1, wobei das nicht-steroidale entzündungshemmende Mittel zu der Familie der Oxycame gehört, vorzugsweise ausgewählt aus Meloxicam, Piroxicam oder Tenoxicam und insbesondere Meloxicam ist.

3. Zusammensetzung gemäß einem der vorhergehenden Ansprüche, wobei die Zusammensetzung einem nicht-humanen Säugetier, insbesondere einem Haus- oder Nutztier, injiziert werden soll.

4. Zusammensetzung gemäß Anspruch 3, **dadurch gekennzeichnet, dass** besagtes Tier ein Rind, ein Schwein, ein Schaf, ein hundeartiges oder ein katzenartiges Tier und insbesondere ein Rind ist.

5. Zusammensetzungen gemäß Anspruch 3, wobei die Injektion eine intramuskuläre oder subkutane Injektion ist.

6. Zusammensetzung gemäß einem der Ansprüche 1 und 2, für eine Anwendung in der Behandlung von Störungen, die mit mikrobiellen Infektionen nicht-humaner Säugetiere verbunden sind, insbesondere durch Injektion besagter Zusammensetzung, wobei die Störungen, die mit mikrobiellen Infektionen verbunden sind, respiratorische Störungen oder Erkrankungen sind, die die oberen oder unteren Atemwege befallen.

7. Kit zur tiermedizinischen Verwendung umfassend eine Zusammensetzung, wie gemäß einem der Ansprüche 1 bis 6 definiert, und wenigstens ein Kompartiment für eine gegebenenfalls sterile Verpackung.

## Claims

1. A parenteral veterinary composition comprising a non-steroidal anti-inflammatory drug, an antibiotic which is florfenicol, at least 35% expressed in g:100ml of dimethylsulphoxide, and at least one polar aprotic solvent which is glycerol formal.

2. The composition according to claim 1, wherein the non-steroidal anti-inflammatory drug is of the oxicam class, preferably selected from meloxicam, piroxicam or tenoxicam, and in particular meloxicam.

3. The composition according to anyone of the preceding claims, said composition being suitable for injection into a non-human mammal, in particular a domestic animal or a farm animal.

4. The composition according to claim 3, wherein said animals are cattle, pigs, sheep, canines or felines, and in particular cattle.

5. The composition according to claim 3, wherein said injection is an intramuscular or subcutaneous injection.

6. The composition according to one of claim 1 and 2, for a use in the treatment of disorders connected with microbial infections of non-human mammals, in particular by injecting the said composition, wherein the disorders connected with microbial infections are respiratory disorders or diseases that affect the lower or upper respiratory tract.

7. A kit for veterinary use comprising a composition as defined in anyone of claims 1-6 and at least one compartment for optionally sterile packaging.
